(19) Europäisches Patentamt
European Patent Office
Office européen des brevets

(11) Numéro de publication: **0 246 975**
**A1**

# (12) DEMANDE DE BREVET EUROPEEN

(21) Numéro de dépôt: **87401141.4**

(22) Date de dépôt: **21.05.87**

(51) Int. Cl.4: **C 12 P 21/04**
C 07 K 7/06, A 61 K 37/02
//(C12P21/04,C12R1:465)

(30) Priorité: **22.05.86 FR 8607269**

(43) Date de publication de la demande:
**25.11.87 Bulletin 87/48**

(84) Etats contractants désignés:
**AT BE CH DE ES FR GB GR IT LI LU NL SE**

(71) Demandeur: **RHONE-POULENC SANTE**
**Les Miroirs 18 Avenue d'Alsace**
**F-92400 Courbevoie Cédex (FR)**

(72) Inventeur: **Deschamps, Maurice**
**3 rue Grévin**
**F-94100 Saint Maur (FR)**

**Floc'h, François**
**70 avenue des Charmes**
**F-94520 Perigny (FR)**

**Jung, Gérard**
**12 rue des Grands Jardins Leuville-sur-Orge**
**F-91310 Montlhery (FR)**

**Margraff, Rodolphe**
**16 allée du Potager**
**F-91170 Viry Chatillon (FR)**

(74) Mandataire: **Pilard, Jacques et al**
**RHONE-POULENC INTERSERVICES Service Brevets**
**Pharma 25, Quai Paul Doumer**
**F-92408 Courbevoie Cédex (FR)**

Revendications pour les Etats contractants suivants: AT + ES + GR.
Le (Les) microorganisme(s) a (ont) été déposé(s) auprès le Centraalbureau voor Schimmelcultures sous le(s) numéro(s) CBS 162.86.

(54) **Nouvelles substances immuno-suppressives, leur préparation par culture de streptomyces sp. (CBS 162.86) et les compositions pharmaceutiques qui les contiennent.**

(57) Nouvelles substances immuno-suppressives désignées par les numéros 55185 RP et 59451 RP, leur préparation par culture de Streptomyces sp. S-16328 (CBS 162.86) et les compositions pharmaceutiques qui les contiennent, et répondant à la formule générale :

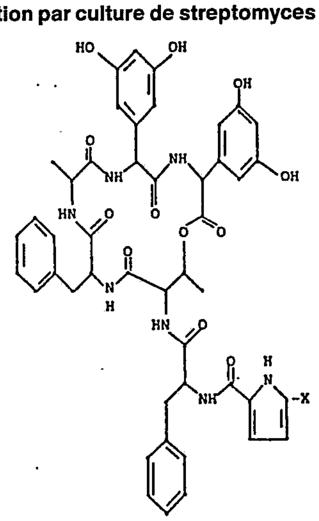

dans laquelle X représente un atome de chlore (55185 RP) ou un atome d'hydrogène (59451 RP).

EP 0 246 975 A1

## Description

NOUVELLES SUBSTANCES IMMUNO-SUPPRESSIVES, LEUR PREPARATION PAR CULTURE DE STREPTOMYCES SP. (CBS 162.86) ET LES COMPOSITIONS PHARMACEUTIQUES QUI LES CONTIENNENT

La présente invention concerne de nouvelles substances immuno-suppressives, désignées ci-après par les numéros 55185 RP et 59451 RP, leur procédé de préparation et les compositions qui les contiennent.

Les 55185 RP et 59451 RP peuvent être obtenus à partir des milieux de culture appropriés d'un nouveau microorganisme identifié plus complètement ci-après, appartenant au genre Streptomyces et désigné par l'appellation Streptomyces sp. S-16328 (CBS 162.86).

Les 55185 RP et 59451 RP sont des cyclodepsipeptides qui répondent à la formule générale :

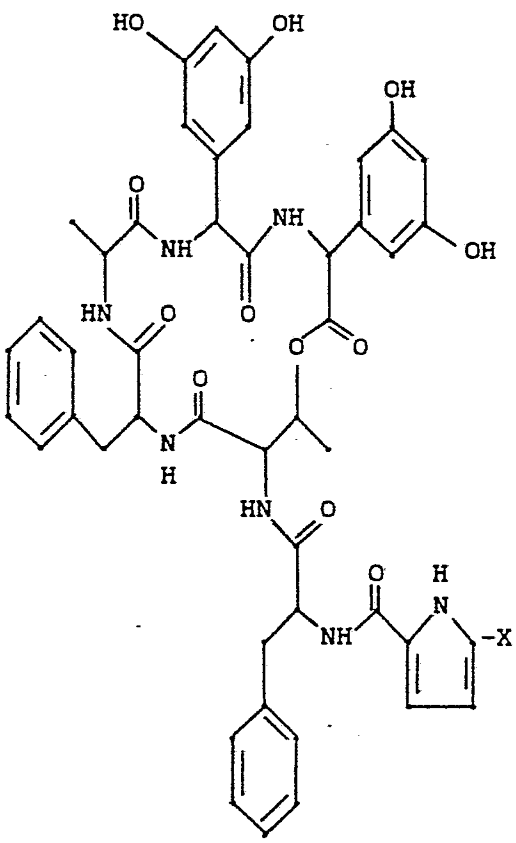

dans laquelle X est un atome de chlore (55185 RP) ou d'hydrogène (59451 RP).

Le 55185 RP est caractérisé par les propriétés physicochimiques suivantes :

- aspect : poudre amorphe blanche à jaune pâle
- solubilité : soluble dans le méthanol, l'éthanol, l'acétate d'éthyle, l'acétone, l'acétonitrile, le diméthylformamide, le diméthylsulfoxyde, insoluble dans l'eau (0,1 à 0,3 ‰ quel que soit le pH), l'éther éthylique et l'hexane.
- composition centésimale : le 55185 RP répond à la formule brute $C_{46}H_{46}ClN_7O_{12}$. La composition centésimale est :

| | calculé | trouvé |
|---|---|---|
| C % | 58,47 | 58,29 |
| H % | 4,58 | 5,30 |
| Cl % | 3,84 | 3,84 |
| N % | 10,61 | 10,13 |
| O % | 22,50 | 22,57 |

- point de fusion : supérieur à 300°C
- pouvoir rotatoire : $[\alpha]_D^{20} = +20,5° \pm 1,4°$ (c = 0,5 ; méthanol)

La structure du 55185 RP a été déterminée à partir de ses spectres ultra-violet, infra-rouge, de masse et de résonance magnétique nucléaire du proton et du $^{13}C$.

- spectre ultra-violet : (détermination à partir d'une solution méthanolique à 267,5 µg/ml sous 1 mm d'épaisseur)

$\lambda$ max = 274 nm ($E^{1\%}_{1cm}$ = 219, $\varepsilon$ = 20240)

On observe un épaulement à 230 nm.

Le spectre ultra-violet n'est pas modifié par addition d'acide chlorhydrique. Après addition de soude, on observe un déplacement bathochrome de 230 à 247 nm et de 274 à 295 nm.

Le spectre ultra-violet du 55185 RP est représenté par la figure 1.

- spectre infra-rouge : (détermination à partir de comprimés en mélange avec KBr)

Le spectre infra-rouge est représenté par la figure 2 dans laquelle sont portés en abscisses les nombres d'ondes en $cm^{-1}$ et en ordonnées les densités optiques.

Dans le tableau I sont indiquées les principales bandes d'absorption infra-rouge du produit exprimées en nombre d'ondes ($cm^{-1}$).

TABLEAU I

| | | | | | |
|---|---|---|---|---|---|
| 3380 | tF (dont $H_2O$) | 1520 | tF | 980 | ép |
| 3300 | tF | 1500 | ép | 950 | f |
| 3090 | f | 1455 | m | 925 | tf |
| 3060 | f | 1440 | ép | 900 | tf |
| 3030 | ép | 1425 | ép | 850 | m |
| 2980 | f | 1405 | f | 795 | ép |
| 2940 | f | 1380 | f | 785 | m |
| 2880 | ép | 1340 | m | 750 | m |
| 2700 | ép | 1310 | m | 700 | m |
| 2500 | ép | 1285 | f | 690 | ép |
| 2340 | $CO_2$ | 1215 | m | 635 | tf |
| 2160 | tf | 1205 | ép | 625 | tf |
| 1950 | ép | 1160 | F | 605 | ép |
| 1880 | tf | 1120 | ép | 580 | m |
| 1740 | F | 1080 | tf | 525 | f |
| 1680 | ép | 1060 | ép | 510 | f |
| 1655 | tF | 1050 | f | 495 | ép |
| 1625 | m | 1030 | tf | 455 | ép |
| 1605 | m | 1010 | ép | 400 | ép |
| 1555 | ép | 1000 | m | 355 | tf |

tF = très forte F = forte

m = moyenne f = faible

tf = très faible ép = épaulement

- spectre de masse :

En ionisation FAB (bombardement par atomes rapides avec un faisceau d'atomes de xénon de 8 keV) avec pour matrice un mélange glycérol-thioglycérol et dans un domaine de masse compris entre 400 et 1000 uma, le

pic de base est l'ion pseudo-moléculaire MH+ = 924 avec un massif isotopique caractéristique de la présence d'un atome de chlore.

Les ions observés aux masses M/z = 650, M/z = 567 et M/z - 506 correspondent aux fragments ci-dessous :

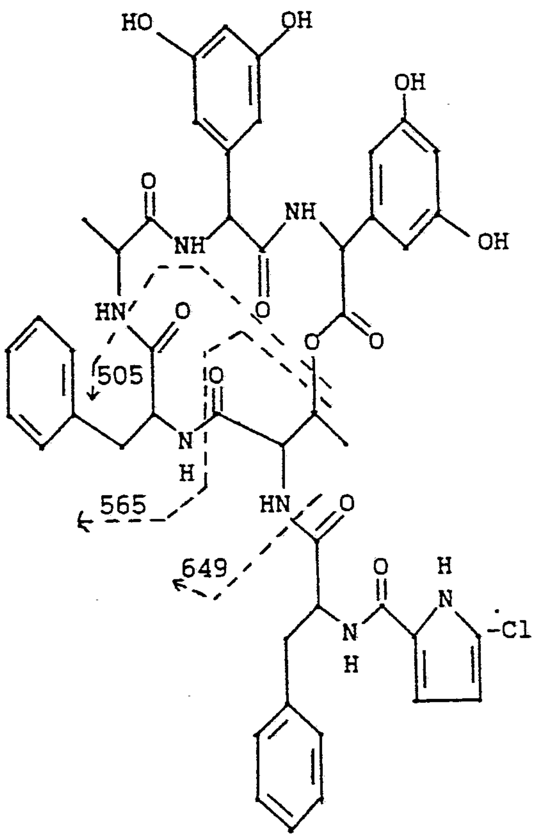


En désorption/ionisation chimique (DCI) avec $NH_3$ comme gaz réactant, on observe le dernier pic à M/z = 880 (MH+ - $CO_2$). Les fragments de masse 275 et 753 correspondent aux structures suivantes :

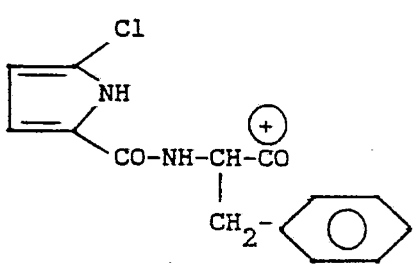

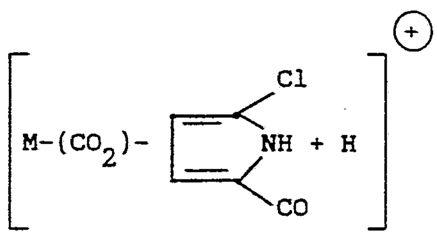

En impact électronique (EI) (bombardement électronique à 70 eV), on observe le dernier pic à la masse M/z = 521. Les fragments de masse M/z = 91 et M/z = 120 sont caractéristiques de la présence de phénylalanine (F).

- spectre de résonance magnétique nucléaire du proton et du $^{13}C$.

Les spectres ont été enregistrés dans le DMSO $D_6$ en opérant à 400,13 MHz pour le proton et à 100,6 MHz pour le carbone et à 40°C.

Les attributions sont données en ppm par rapport à la raie central du DMSO (2,5 ppm pour le proton ; 39,5 ppm pour le carbone).

L'analyse du spectre de résonance magnétique nucléaire du proton est donnée dans le tableau II.

TABLEAU II

| | Hα | Hβ | Hγ | NH | Aromatique | OH |
|---|---|---|---|---|---|---|
| T | 4,45 (m) | 4,98 (m) | 1.15 (d)<br>J = 6 Hz | 8.6 (d)<br>J = 9 Hz | - | - |
| A | 4.00 (m) | 1.38 (d)<br>J = 7 Hz | - | 8.16 (d)<br>J = 5 Hz | - | - |
| $F_1$ | 4.80 (m) | 2.80 )<br>3.10 ) (m) | - | 8.09 (d)<br>J = 9 Hz | 7.05 )<br>7.30 ) (m) | - |
| $F_2$ | 4.55 (m) | 2.90 )<br>3.15 ) (m) | - | 8.55 (d)<br>J = 9 Hz | 7.05 )<br>7.30 ) (m) | - |
| $FG_1$ | 5.45 (d)<br>J = 9 Hz | - | - | 7.80 (d)<br>J = 9 Hz | 6.22 )<br>6.28 ) (s) | 9.23 (bs) |
| $FG_2$ | 5.25 (d)<br>J = 8 Hz | - | - | 8.27 (d)<br>J = 8 Hz | 6.15 )<br>6.18 ) (s) | 9.14 (bs) |
| ClP | - | - | - | 12,1 (s) | 6.05 & 6.90<br>2 d J = 3.5 Hz | - |

La constante de couplage J est exprimée en Hz
d = doublet
bs = broad singulet
T = thréonine
F = phénylalanine
ClP = chloro-pyrrole
s = singulet
m = multiplet
A = alanine
FG = phénylglycine

Les résultats concernant les attributions du $^{13}C$ sont les suivants :

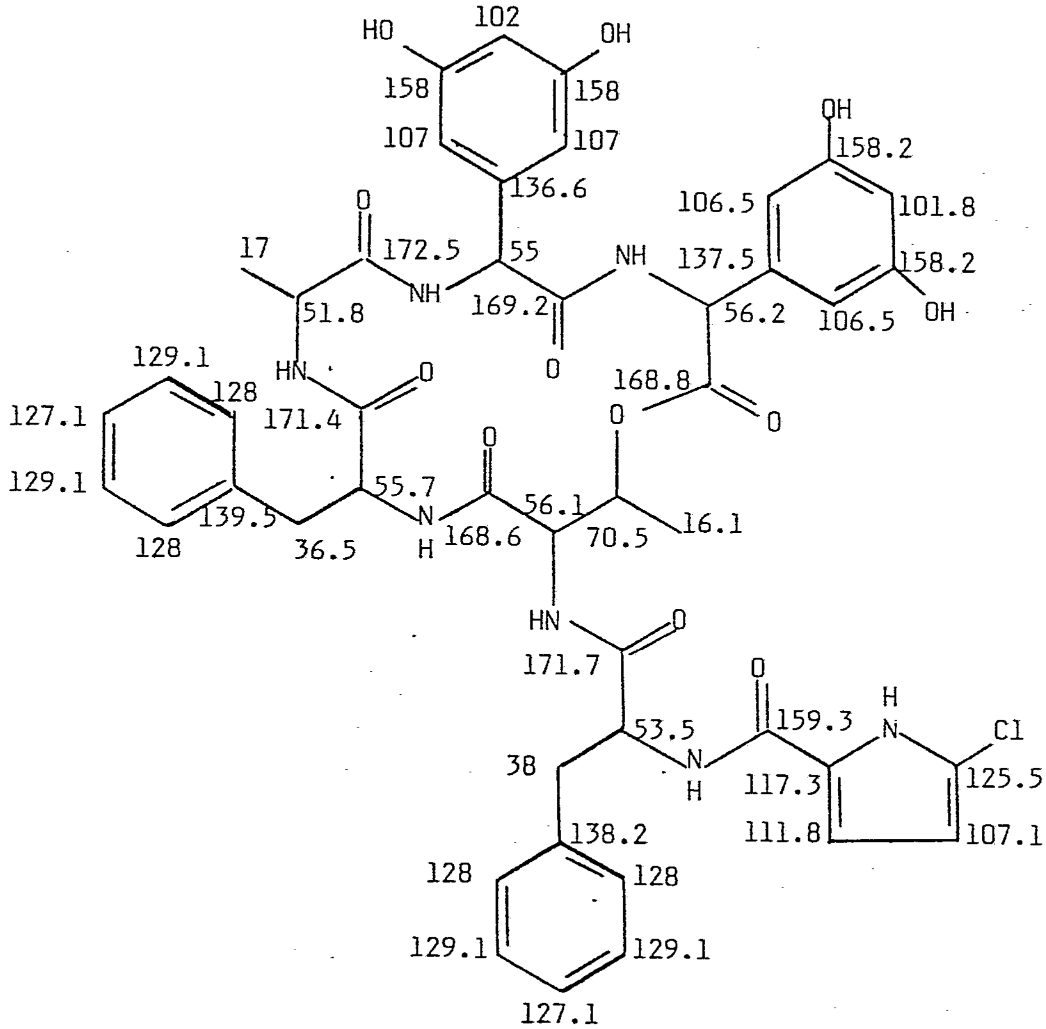

Par chromatographie ascendante sur couche mince de gel de silice, en utilisant le mélange solvant dichloro-1,2 éthane-méthanol (80-20 en volumes), le Rf est voisin de 0,5. Sur plaque de silice silanisée MERCK, en utilisant le mélange solvant acétonitrile-eau contenant 3 % de NaCl (40-60 en volumes), le Rf est voisin de 0,14 (révélation en orangé avec le réactif d'Ehrlich).

Le 59451 RP est caractérisé par les propriétés physicochimiques suivantes :
- aspect : poudre amorphe blanche à jaune pâle
- solubilité : soluble dans le méthanol, l'éthanol, l'acétate d'éthyle, l'acétone, l'acétonitrile, le diméthylformamide, le diméthylsulfoxyde, insoluble dans l'eau (0,1 à 0,3 % quel que soit le pH), l'éther éthylique et l'hexane
- composition centésimale : le 59451 RP répond à la formule brute $C_{46}H_{47}N_7O_{12}$. La composition centésimale est :

| | calculé | trouvé (tel quel) |
|---|---|---|
| C % | 62,09 | 58,64 |
| H % | 5,32 | 5,42 |
| N % | 11,02 | 10,47 |
| O % | 21,57 | 25,10 |
| $H_2O$ % | - | 5,37 |

- point de fusion : 275-280°C (décomposition)
- pouvoir rotatoire : $[\alpha]_D^{20}$ = +24,3° ± 1° (c = 0,5, méthanol).

La structure du 59451 RP a été déterminée à partir de ses spectres ultra-violet, infra-rouge, de masse et de résonance magnétique nucléaire du proton et du $^{13}C$.

- spectre ultra-violet (détermination à partir d'une solution méthanolique à 38 µg/ml sous 1 cm d'épaisseur)
  $\lambda$ max = 268 nm ($E^{1\%}_{1\,cm}$ = 223, $\varepsilon$ = 19864)

Le spectre ultra-violet du 59451 RP est représenté par la figure 3.

- spectre infra-rouge (détermination à partir de comprimés en mélange avec KBr).

Le spectre infra-rouge est représenté par la figure 4 dans laquelle sont portés en abscisses les nombres d'ondes en $cm^{-1}$ et en ordonnées les densités optiques.

Dans le tableau III sont indiquées les principales bandes d'absorption infra-rouge du 59451 RP exprimées en nombre d'ondes ($cm^{-1}$).

TABLEAU III

| | | |
|---|---|---|
| 3360 ép | 1455 m | 920 f |
| 3480 ép (dont $H_2O$) | 1445 ép | 900 tf |
| 3400 tF | 1415 m | 885 tf |
| 3280 tF | 1380 m | 870 ép |
| 3060 ép | 1340 m | 850 m |
| 3040 ép | 1310 m | 830 ép |
| 2980 f | 1295 ép | 785 ép |
| 2940 f | 1275 ép | 750 ép |
| 2880 ép | 1245 tf | 740 F |
| 2680 ép | 1230 ép | 700 F |
| 2520 ép | 1190 ép | 690 ép |
| 2160 ép | 1170 F | 645 tf |
| 2060 ép | 1145 ép | 620 tf |
| 1950 ép | 1110 m | 605 ép |
| 1880 ép | 1090 ép | 585 m |
| 1740 ép | 1070 f | 550 ép |
| 1675 ép | 1060 ép | 520 tf |
| 1655 ép | 1040 f | 505 m |
| 1640 tF | 1030 ép | 470 tf |
| 1625 tF | 1000 m | 445 tf |
| 1605 F | 990 tf | 400 ép |
| 1555 F | 970 ép | 330 ép |
| 1530 F | 960 tf | |
| 1500 ép | 930 ép | |

tF = très forte
F = forte
m = moyenne

tf = très faible
f = faible
ép = épaulement

- spectre de masse

En ionisation FAB (bombardement par atomes rapides avec un faisceau d'atomes de xénon de 8 keV) avec pour matrice un mélange glycérol-thioglycérol, le pic de base est l'ion pseudo-moléculaire MH+ = 890.

On observe des ions aux masses M/z = 781 et M/z =650.

L'ion observé à la masse M/z = 650 correspond au fragment :

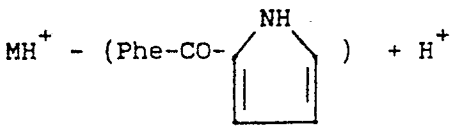

En désorption/ionisation chimique (DCI) avec $NH_3$ comme gaz réactant, on obtient les fragments de masse M/z = 241 et M/z = 341 qui correspondent aux structures suivantes :

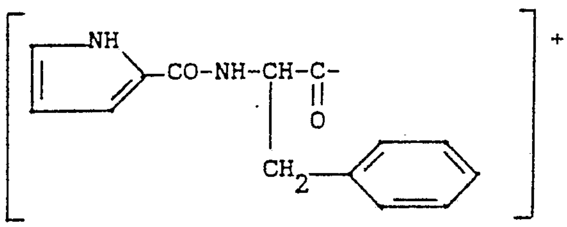

et

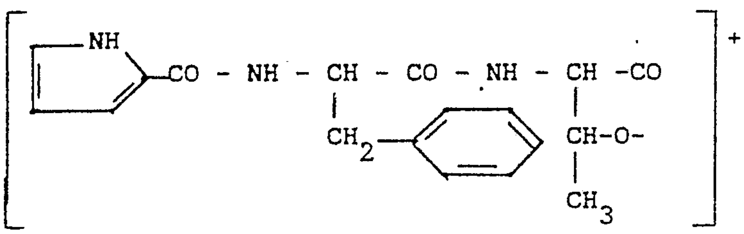

- spectre de résonance magnétique nucléaire du proton et du $^{13}C$.

Les spectres ont été enregistrés dans le DMSO $D_6$ en opérant à 250 MHz pour le proton et à 100,6 MHz pour le carbone.

Les attributions sont données en ppm par rapport à la raie centrale du DMSO (2,5 ppm pour le proton ; 39,5 ppm pour le carbone).

L'analyse du spectre de résonance magnétique nucléaire du proton est donnée dans le tableau IV.

TABLEAU IV

| | Hα | Hβ | Hγ | NH | Aromatique | OH |
|---|---|---|---|---|---|---|
| T | 4,5 (m) | 5 (m) | 1.10 (d)<br>J = 6 Hz | 8.75 (d)<br>J = 8 Hz | - | - |
| A | 3.95 (m) | 1.35 (d)<br>J = 7 Hz | - | 8.25 (d)<br>J = 5 Hz | - | - |
| $F_1$ | 4.8 (m) | 3 (m) | - | 8.15 (d)<br>J = 9 Hz | 7 à 7,40 | - |
| $F_2$ | 4.5 (m) | 3 (m) | - | 8.75 (d)<br>J = 8 Hz | 7 à 7,40 | - |
| $FG_1$ | 5.45 (d)<br>J = 9 Hz | - | - | 7.85 (d)<br>J = 9 Hz | 6,2 à 6,3 | 9.35 (bs) |
| $FG_2$ | 5.25 (d)<br>J = 8 Hz | - | | 8.35 (d)<br>J = 8 Hz | 6,2 à 6,3 | 9.25 (bs) |
| P | - | - | - | 11,45 (bs) | 6,95 ; 6,85 et 6,10<br>(3 x m) | - |

La constante de couplage J est exprimée en Hz

d = doublet

bs = broad singulet

T = thréonine

F = phénylalanine

P = pyrrole

s = singulet

m = multiplet

A = alanine

FG = phénlglycine

Le spectre de résonance magnétique nucléaire du $^{13}C$ du 59451 RP est identique à celui du 55185 RP à ± 0,2 ppm près à l'exception de la partie pyrrole :

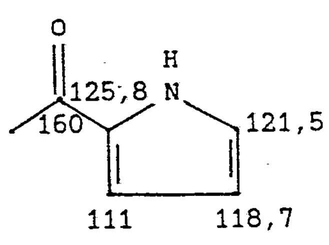

Par chromatographie ascendante sur couche mince de gel de silice, en utilisant le mélange solvant dichloro-1,2 éthane-méthanol (80-20 en volumes), le Rf est voisin de 0,5. Sur plaque de silice silanisée MERCK, en utilisant un mélange solvant acétonitrile-eau contenant 3 % de NaCl (40-60 en volumes), le Rf est voisin de 0,21 (révélation en rouge brique avec le réactif d'Ehrlich).

Les 55185 RP et 59451 RP peuvent être caractérisés par les réactions colorées avec différents réactifs. Ils donnent des réactions positives avec l'iode, le réactif de Gibbs, la vanilline sulfurique, le chlorure ferrique, le réactif de Greig-Leaback (chlore-tolidine) et le réactif d'Ehrlich. Ils donnent des réactions négatives avec la ninhydrine et le réactif de Dragendorff.

L'organisme producteur des 55185 RP et 59451 RP est une souche d'Actinomycétale qui a été isolée à partir d'un échantillon de terre prélevé en Espagne à Escandon auquel a été attribué le numéro S-16328. Un échantillon de ce microorganisme a été déposé au Centraalbureau voor Schimmelcultures à Baarn (Pays-Bas) où il a été enregistré sous la référence CBS 162.86.

Cet organisme, présentant des caractères qui n'ont pas permis de l'identifier à une espèce déjà décrite, doit être considéré comme une espèce nouvelle et il a été désigné par l'appellation Streptomyces sp. S-16328.

L'isolement a été effectué en suivant la méthode générale qui consiste à mettre une petite quantité de terre en suspension dans de l'eau distillée stérile, à diluer la suspension à différentes concentrations et à étaler un petit volume de chaque dilution sur la surface de la boîte de Pétri contenant un milieu nutritif gélosé. Après une incubation de quelques jours à 26° C, qui permet aux microorganismes de se développer, les colonies que l'on veut isoler pour en poursuivre l'étude sont prélevées et repiquées sur des géloses nutritives afin d'en obtenir des cultures plus abondantes.

L'Actinomycetale S-16328 appartient à la famille des Streptomycetaceae genre Streptomyces car sa paroi cellulaire contient de l'acide L diamino-2,6 pimélique.

Le Streptomyces S-16328 forme des spores épineuses cylindriques mesurant 0,4 à 0,6 µm/l à 1,2 µm. Ses chaînes sporifères droites ou flexueuses sont longues et comportent le plus souvent plusieurs dizaines de spores. Les sporophores sont simples. Par son mode de sporulation, cette souche se classe dans la Section RectusFlexibilis de la classification de Pridham.

Le Streptomyces S-16328 présente un mycélium aérien sporulé de couleur beige rosé. Il se développe bien à 28° C. La coloration de son mycélium végétatif passe en général, selon le milieu de culture, du blanc jaunâtre au marron beige.

Le Streptomyces ne donne pas de pigment soluble sur les milieux sur lesquels il a été observé.

Dans ces cultures effectuées à 28° C, il présente les caractères biochimiques suivants :

- production de mélanine négative
- production de $H_2S$ négative
- tyrosinase postive
- hydrolyse de la caséine positive
- hydrolyse de la gélatine positive
- production de nitrites à partir de nitrates négative
- hydrolyse de l'amidon positive
- culture sur lait peptonisation sans coagulation avec alcalinisation du pH qui passe de 6,4 à 7,5 en 28 jours

Les caractères culturaux du Streptomyces sont rassemblés dans le tableau V. Ce sont ceux de cultures arrivées à un bon stade de dévoloppement, c'est-à-dire de 2 à 3 semaines à 28° C. Ces caractères ont été observés sur des géloses nutritives et des bouillons habituellement utilisés pour déterminer les caractères morphologiques des souches de Streptomyces.

1 Les cultures sur milieux gélosés ont été effectuées en boîte de Pétri, excepté pour les milieux $ISP_6$, $ISP_7$ et mélanine de Waksman, où elles ont été réalisées sur des géloses inclinées. Les références ou constitutions des milieux de culture sont les suivantes :

Réf. 1 : "Methods for characterization of Streptomyces species", International Journal of Systematic Bacteriology -E.B. Shirling and D. Gottlieb, vol. 16, no 3, 1966 p. 313-340

Réf. 2 : "Melanin formation medium" S.A. Waksman, The Actinomycetes, vol. 2, no 42, p. 333, The Williams and Wilkins Company, Baltimore, 1961

Réf. 3 : "Manual of Methods for Pure Culture Study of Bacteria", Society of American Bacteriologists, Geneva, N.Y. $II_{50}$-18

Réf. 4 : "The Taxonomy of soil Bacteria" - R.E. Gordon, Ecology of Soil Bacteria, Liverpool University Press 1967, T.R.G. Gray, B. Parkinson Ed.

Réf. 5 : "Plain Gelatin" préparé suivant les indications du "Manual of Methods for Pure Culture Study of Bacteria", Society of American Bacteriologists, Geneva, N.Y. $II_{50}$-19

Réf. 6 : Lait écrémé en poudre commercial reconstitué selon les indications du fabricant.

TABLEAU V

| Milieu de culture | Degré de développement | Mycélium végétatif ou envers de la culture | Appareil aérien (comprenant l'ensemble du mycélium aérien et de la sporulation) | Pigment soluble | Observations et propriétés biochimiques |
|---|---|---|---|---|---|
| $ISP_1$ (réf. 1) | bon | blanc jaunâtre plissé-abondant | 0 | 0 | - |
| $ISP_2$ (réf. 1) | bon | marron beige plissé-abondant | blanchâtre à l'état de traces spores = 0 | 0 | - |
| $ISP_3$ (réf. 1) | moyen | blanc grisâtre | 0 | 0 | - |
| $ISP_4$ (réf. 1) | très bon | beige jaunâtre plissé | beige rosé spores nombreuses | 0 | utilisation de l'amidon |
| $ISP_5$ (réf. 1) | faible | beige grisâtre plissé | 0 | 0 | - |
| $ISP_6$ (réf. 1) | assez bon | blanchâtre abondant plissé | 0 | 0 | production de $H_2S$ négative |
| $ISP_7$ (réf. 1) | assez bon | plissé blanc jaunâtre | 0 | léger pigment soluble brun rose | production mélanine négative |
| gélose mélanine de Waksman (réf.2) | pauvre | blanchâtre léger | 0 | 0 | production mélanine négative |

TABLEAU V

| Milieu de culture | | Degré de développement | Mycélium végétatif ou envers de la culture | Appareil aérien (comprenant l'ensemble du mycélium aérien et de la sporulation) | Pigment soluble | Observations et propriétés biochimiques |
|---|---|---|---|---|---|---|
| bouillon nutritif nitrate Difco (réf.3) | | moyen | culture floconneuse blanchâtre | 0 | 0 | formation de nitrite négative |
| gélose à la caséine (réf. 4) | | bon | blanc jaunâtre à brun jaunâtre abondant et plissé | 0 | 0 | hydrolyse de la caséine très rapide |
| gélose à la tyrosine | | bon | blanc jaunâtre plissé | blanchâtre, développement très faible | 0 | dégradation de la tyrosine très rapide |
| culture sur gélatine pure à 12 % (réf. 5) | | moyen | blanchâtre | 0 | 0 | liquéfaction de la gélatine positive et totale après 7 jours de culture |
| Lait écrémé (réf. 6) | 28°C | bon | anneau jaunâtre bien développé | blanc peu abondant | | peptonisation sans coagulation pH passant de 6,4 à 7,5 en 1 mois (témoin 6,25 6,20) |
| | 37°C | bon | anneau brunâtre assez bien développé | 0 | | peptonisation sans coagulation pH passant de 6,43 à 6,80 en 1 mois (témoin pH 6,16 6,11) |

Le procédé de préparation des 55185 RP et 59451 RP consiste essentiellement à cultiver Streptomyces sp. S-16328 ou ses mutants producteurs sur un milieu et dans des conditions appropriées et à séparer le produit

formé au cours de la culture.

La culture de Streptomyces sp. S-16328 peut être effectuée par toute méthode de culture aérobie en surface ou en profondeur mais cette dernière est à préférer pour des raisons de commodité. On utilise à cette fin les différents types d'appareils qui sont d'un usage courant dans l'industrie des fermentations.

On peut en particulier adopter la marche suivante pour la conduite des opérations :

Streptomyces sp. S-16328 - stock

↓

Culture sur gélose

↓

Culture en fiole agitée

↓

Culture inoculum en fermenteur

↓

Culture de production en fermenteur

Le milieu de fermentation doit contenir essentiellement une source de carbone et une source d'azote assimilables, des éléments minéraux, en particulier des chlorures, et éventuellement des facteurs de croissance, tous ces éléments pouvant être apportés sous forme de produits bien définis ou par des mélanges complexes, tels qu'on en rencontre dans des produits biologiques d'origines diverses.

Comme sources de carbone assimilable, on peut utiliser des hydrates de carbone tels que le glucose, la saccharose, le maltose, les dextrines, l'amidon ou d'autres substances hydrocarbonées comme des sucres alcools (glycérol) ou comme certains acides organiques : acides lactique, citrique. Certaines huiles animales ou végétales comme l'huile de lard ou l'huile de soja peuvent remplacer avantageusement ces différentes sources hydrocarbonées ou leur être adjointes.

Les sources convenables d'azote assimilable sont extrêmement variées. Elles peuvent être des substances chimiques simples comme les sels minéraux ou organiques d'ammonium, l'urée, certains acides aminés. Elles peuvent aussi être apportées par des substances complexes contenant principalement de l'azote sous forme protidique : caséine, lactalbumine, gluten et leurs hydrolysats, farine de soja, d'arachide, de poisson, extraits de viande, de levure, solubles de distillerie, corn-steep.

Parmi les éléments minéraux ajoutés, certains peuvent avoir un effet tampon ou neutralisant comme les phosphates alcalins ou alcalino-terreux ou les carbonates de calcium et de magnésium. D'autres apportent l'équilibre ionique nécessaire au développement de Streptomyces sp. S-16328 et à l'élaboration des 55185 RP et 59451 RP comme les chlorures et sulfates de métaux alcalins ou alcalino-terreux. Enfin certains agissent plus spécialement comme activateurs des réactions métaboliques de Streptomyces sp. S-16328, ce sont les sels de zinc, de cobalt, de fer, de cuivre, de manganèse.

Les facteurs de croissance sont des produits de nature vitaminique tels que la riboflavine, l'acide folique, l'acide pantothénique.

Le pH du milieu de fermentation au départ de la culture doit être compris entre 5,8 et 7,8 et de préférence entre 6,2 et 7,4. La température optimale pour la fermentation est comprise entre 25 et 30°C, mais une production satisfaisante est obtenue pour des températures comprises entre 23 et 33°C. L'aération de la fermentation peut varier dans de larges limites. On a cependant trouvé que des aérations de 0,3 à 3 litres d'air par litre de bouillon et par minute conviennent particulièrement bien. Le rendement maximal en 55185 RP est obtenu après 2 à 8 jours de culture, ce temps dépendant essentiellement du milieu utilisé.

D'après ce qui précède, on conçoit que les conditions générales de la culture de Streptomyces sp. S-16328 pour la production de 55185 RP et 59451 RP peuvent varier dans une large mesure et être adaptées à chaque nécessité particulière.

Les 55185 RP et 59451 RP peuvent être isolés des moûts de fermentation de la manière suivante :

Le moût est filtré à un pH généralement comprise entre 6 et 8, de préférence voisin de 7 en présence d'un adjuvant de filtration.

L'activité retenue dans le gâteau de filtration en est extraite à l'aide d'un solvant organique approprié, une cétone telle que l'acétone ou un alcool tel que le méthanol. Le produit brut peut être isolé à partir des solutions organiques par cristallisation après concentration de ces solutions sous pression réduite, addition éventuelle d'un mauvais solvant ou d'un non solvant et séjour en chambre froide.

Les 55185 RP et 59451 RP peuvent être séparés et purifiés par des méthodes habituelles telles que la recristallisation, la chromatographie sur divers supports adsorbants ou la distribution à contre-courant.

Le 59451 RP peut également être obtenu par déchloration du 55185 RP selon les méthodes habituelles qui permettent de remplacer un atome de chlore par un atome d'hydrogène sans toucher au reste de la molécule.

Il est particulièrement avantageux de traiter le 55185 RP en solution méthanolique par l'hydrogène gazeux

en présence d'un catalyseur à une température voisine de 20° C. Comme catalyseur on utilise généralement le palladium sur charbon en présence d'un accepteur d'acide tel que la magnésie.

Les exemples suivants, donnés à titre non limitatif, montrent comment l'invention peut être mise en pratique.

EXEMPLE 1 (Fermentation)

Dans un fermenteur de 170 litres, on charge :
- peptone 1200 g
- extrait de levure 600 g
- cérélose 1200 g
- chlorure de sodium 600 g
- eau de ville, complément pour 120 litres

Le pH est ajusté à 7,0 par addition de 30 cm3 de soude 10N.

On stérilise le milieu par barbotage de vapeur à 122° C pendant 40 minutes. Après refroidissement le pH du milieu est de 6,6.

On ensemence avec 200 cm3 d'une culture en erlenmeyer agitée pendant 72 heures de Streptomyces sp. S-16328.

La culture est développée à 28° C pendant 36 heures en agitant à 250 tours/minute et en aérant avec un volume d'air stérile au débit de 5 m3/heure. Elle est alors convenable pour l'ensemencement de la culture productrice.

La culture productrice est effectuée dans un fermenteur de 800 litres dans lequel on a chargé les substances suivantes :
- solubles de distillerie stérilisés 10 kg
- cérélose stérilisé 4 kg
- huile de soja 2 litres
- carbonate de calcium 2 kg
- sulfate d'ammonium stérilisé 0,8 kg
- eau de ville, complément pour 400 litres

Le pH est ajusté à 7,0 par addition de 440 cm3 de soude 10N.

On stérilise le milieu par barbotage de vapeur à 122° C pendant 40 minutes après avoir chauffé pendant 15 minutes à 100° C.

Le pH du milieu est alors de 6,75.

On ensemence alors avec 40 litres de la culture inoculum en fermenteur de 170 litres décrite ci-dessus.

La culture est développée à 26° C pendant 83 heures en agitant à 250 tours/minute et en aérant avec un volume d'air stérile au débit de 20 m3/heure.

En fin d'opération, le pH de la culture est de 8,05 et le volume du moût est de 410 litres.

EXEMPLE 2 (Extraction-Purification)

On filtre 400 litres du moût de fermentation en présence de 20 kg d'adjuvant de filtration (Clarcel) sur filtre-presse. Le gâteau de filtration renfermant le mycélium est délité, sous forte agitation, dans 200 litres d'acétone contenant 20 % d'eau. On filtre sur filtre-presse en présence d'adjuvant de filtration. Le filtrat obtenu est concentré sous pression réduite pour éliminer l'acétone. La solution aqueuse résiduelle est extraite à un pH voisin de 7 par 2 fois 50 litres d'acétate d'éthyle. La phase organique est séparée par décantation puis concentrée à sec sous pression réduite (2-3 torrs ; 0,27-0,4 kPa). On obtient ainsi 183 g d'un résidu huileux.

Le résidu huileux est dissous dans 1,5 litre de méthanol puis déposé, par évaporation sous pression réduite (2-3 torrs ; 0,27-0,4 kPa) sur 500 cm3 de résine "Duolite S 861". La résine est chargée au sommet d'une colonne de 7,5 cm de diamètre contenant 3 litres de résine "Duolite S 861" équilibrée dans un mélange méthanol-eau (50-50 en volumes). On élue au débit de 640 cm3/heure en fractionnant toutes les 30 minutes et en éluant successivement avec :
- méthanol-eau (50-50 en volumes) : fractions 1 à 34
- méthanol-eau (70-30 en volumes) : fractions 35 à 71
- méthanol-eau (80-20 en volumes) : fractions 72 à 100

Chaque fraction est contrôlée par chromatographie sur couche mince de silicagel en éluant avec le système dichloro-1,2 éthane-méthanol (80-20 en volumes) et en révélant, pour un Rf voisin de 0,5, avec le réactif de Gibbs qui donne une coloration bleue caractéristique avec les composés à fonction phénol.

La majeure partie du 55185 RP se trouve dans les fractions 87 à 97 qui sont réunies puis déposées, par évaporation sous pression réduite (2-3 torrs ; 0,27-0,4 kPa) sur 400 cm3 de silicagel "GRACE 60 Å" ; 40-63 microns).

La poudre sèche obtenue est déposée sur une colonne de 7,5 cm de diamètre et de 110 cm de hauteur de silice équilibrée dans le mélange dichloro-1,2 éthane-méthanol (80-20 en volumes). On effectue une élution isocratique au débit de 600 cm3/heure. Le 55185 RP est élué dans les fractions 6 à 15. Les trois dernières fractions qui renferment un pigment jaune sont éliminées. Après évaporation sous pression réduite (2-3 torrs ; 0,27-0,4 kPa) des fractions 6 à 12 réunies, on obtient 6,8 g d'un solide gommeux beige.

Le produit ainsi obtenu est repris par 50 cm3 de méthanol. La solution est filtrée puis concentrée jusqu'à un

volume de 5 cm3 et enfin déposée sur une colonne de 5 cm de diamètre et de 138 cm de hauteur de Sephadex LH 20 monté dans le méthanol. On élue avec du méthanol pur au débit de 300 cm3/heure en recueillant des fractions toutes les 20 minutes. Les fractions sont contrôlées par chromatographie en couche mince. Les fractions 10 à 13, qui contiennent le 55185 RP sont réunies et concentrées jusqu'à un volume de 100 cm3. On ajoute alors 150 cm3 de cyclohexane. Après agitation puis décantation, la phase méthanolique est concentrée à sec sous pression réduit (2-3 torrs ; 0,27-0,4 kPa). On obtient ainsi 2,32 g de 55185 RP contenant 6 % de 59451 RP.

EXEMPLE 3

On filtre 400 litres du moût de fermentation en présence de 20 kg d'adjuvant de filtration (Clarcel) sur filtre-presse. Le gâteau de filtration contenant le mycélium est délité sous forte agitation, dans 200 litres d'acétone contenant 20 % d'eau. On filtre sur filtre-presse en présence d'adjuvant de filtration. Le filtrat obtenu est concentré sous pression réduite pour éliminer l'acétone. La solution aqueuse résiduelle est extraite à un pH voisin de 7 par 2 fois 50 litres d'acétate d'éthyle. La phase organique est separée par décantation puis concentrée à sec sous pression réduite (2-3 torrs ; 0,27-0,4 kPa). On obtient ainsi un résidu huileux.

Le résidu huileux obtenu est repris par 5 litres de méthanol. On ajoute, sous agitation, 15 litres de cyclohexane. Après décantation, la phase méthanolique est évaporée sous pression réduite et le résidu est dispersé dans 1 litre d'éther éthylique. Il se forme une poudre fine (32,5 g) qui est séparée par filtration.

On reprend la poudre obtenue (26 g) par 500 cm3 de méthanol et la solution obtenue est adsorbée sur 185 g de silicagel (granulométrie : 35-70 microns ; porosité 60 Å). La poudre sèche est chargée dans une colonne de 400 cm3 qui est complétée avec du silicagel. On élue avec 1 litre d'acétate d'éthyle pur. La solution obtenue, après concentration à sec, conduit à 11,7 g d'une poudre jaune.

On reprend 10 g de la poudre obtenue précédemment dans 150 cm3 de méthanol et la solution obtenue est adsorbée sur 100 cm3 de silice greffée (C 18 de GRACE : granulométrie 20 microns ; porosité 100 Å). La poudre sèche obtenue est chargée dans une "pré-colonne" (diamètre 7,5 cm ; hauteur : 10 cm) puis on complète par de la silice greffée. Après fermeture de la "pré-colonne", l'air qu'elle renferme est chassé par un courant d'eau distillée puis l'eau distillée est déplacée par 400 cm3 d'acétonitrile à 40 %.

On raccorde ensuite la "pré-colonne" à une colonne (diamètre 7,5 cm ; hauteur 50 cm) contenant le même support chromatographie équilibré dans l'acétonitrile à 40 %. On effectue une élution isocratique à un débit de 150 cm3/minute en recueillant des fractions de 500 cm3. Les fractions sont contrôlées par chromatographie en couche mince sur plaques de silice silanisée MERCK éluées par un mélange acétonitrile-chlorure de sodium en solution aqueuse à 3 % (40-60 en volumes).

Les produits élués se différencient par leur coloration avec le réactif d'Ehrlich et leur Rf.

Les fractions 12 à 15 renferment 0,46 g d'un produit dont le Rf = 0,21, révélé en rouge brique et qui correspond au 59451 RP.

Les fractions 18 à 51 referment 5,96 g d'un produit dont le Rf = 0,14, révélé en orangé et qui correspond au 55185 RP.

On reprend 1,13 g du produit dont le Rf = 0,21 dans 15 cm3 d'un mélange méthanol-eau (65-35 en volumes).

La solution limpide est injectée sur une colonne (diamètre 5 cm ; longueur 50 cm) de MCI gel CHP 20P équilibré dans le même mélange de solvants. On effectue une élution isocratique à un débit de 17 cm3/minute en recueillant 50 fractions de 100 cm3 puis on élue avec un mélange méthanol-eau (72-28 en volumes) dans les mêmes conditions. On recueille ainsi 60 fractions supplémentaires.

Les fractions 78 à 110 fournissent 0,59 g de 59451 RP pur.

On reprend 10,5 g du produit dont le Rf = 0,14 dans 350 cm3 de méthanol à 65 %. La solution limpide est injectée sur une colonne (diamètre 5 cm ; longueur 50 cm) de MCI gel CHP 20P équilibré dans le méthanol à 65 %. On élue dans les mêmes conditions que précédemment à un débit de 17 cm3/minute avec 4 litres de méthanol à 65 % puis avec 4 litres de méthanol à 85 % en recueillant des fractions de 100 cm3.

Les fractions 46 à 56 fournissent 9,78 g de 55185 RP pur.

EXEMPLE 4

On ajoute 14 g de 55185 RP pur à une suspension de 3,5 g de palladium à 5 % sur charbon et 3,5 g de magnésie (MgO) dans 120 cm3 de méthanol.

On envoie un courant d'hydrogène, bulle à bulle, à une température voisine de 20°C et sous 760 mm de mercure (101,3 kPa) pendant 15 heures.

Une analyse par chromatographie liquide à haute performance montre que la déchloration du 55185 RP en 59451 RP est quantitative.

Le mélange réactionnel est filtré en présence d'adjuvant de filtration (Célite). Après évaporation du filtrat à sec on obtient 15,2 g d'une poudre blanche contenant du méthanol résiduel.

On reprend 11 g de la poudre ainsi obtenue par 150 cm3 de méthanol. La solution est adsorbée sur 60 cm3 de silice greffée (C18 de GRACE N.D.) de granulométrie 35-70 microns.

La poudre sèche obtenue est chargée à sec dans une "pré-colonne" (diamètre 7,5 cm ; hauteur 10 cm) en complétant avec la même silice greffée vierge. Après fermeture de la "pré-colonne", l'air qu'elle contient est chassé par un courant d'eau distillée et cette dernière par 400 cm3 d'acétonitrile à 40 %.

On raccorde ensuite la "pré-colonne" à une colonne de 7,5 cm de diamètre et de 50 cm de hauteur renfermant la même silice greffée C18 mais dont la granulométrie est de 20 microns équilibrée dans l'acétonitrile à 40 %.

On effectue une élution isocratique à un débit de 150 cm3/minute en collectant des fractions de 500 cm3 après passage de 1,3 litre d'éluant.

Les fractions 8 à 12, après évaporation sous pression réduite, fournissent 8,9 g de 59451 RP qui est purifié dans les conditions décrites dans l'exemple 3.

Les 55185 RP et 59451 RP présentent des propriétés immuno-suppressives remarquables. Plus particulièrement, les 55185 RP et 59451 RP sont des immuno-suppresseurs de l'immunité cellulaire.

In vitro, à des concentrations molaires comprises entre $10^{-5}$ et $10^{-8}$, les 55185 RP et 59451 RP inhibent de manière significative :

- l'activité cytostatique de macrophages péritonéaux de souris induits par le thioglycolate et particulièrement s'ils sont stimulés par l'addition d'interleukine-2 aux lymphocytes pendant la phase d'incubation
- la production d'interleukine-2 par les lymphocytes humains stimulés à la phytohemagglutinine (PHA) ou par les thymocytes murins stimulés à la PHA et l'interleukine-1
- la production d'interféron gamma par les lymphocytes humains ou par les thymocytes murins.

In vitro, les 55185 RP et 59451 RP ne manifestent pas d'effet inhibiteur sur la sécrétion d'anticorps [techniques de P.H. Klesius, Proc. Soc. Exp. Biol. Med. (N.Y.), 135, 155 (1970) et de H. Van Dijk et N. Bloksma, J. Immunol. Methods, 14, 325 (1977)] et ils ne sont pratiquement pas cytotoxiques pour les cellules de la leucémie P388.

Les 55185 RP et 59451 RP, administrés à la souris à des doses comprises entre 25 et 50 mg/kg i.p. un jour avant le test inhibent l'activité NK mesurée vis-à-vis des cellules cibles YAC/1.

Administrés par voie orale à la dose de 50 mg/kg pendant 10 jours, les 55185 RP et 59451 RP retardent significativement le rejet de greffe de peau allogénique ($C_{57}$ Bl/6) chez la souris Balb/c, ou semi-allogénique ($B_6D_2F_1$) chez la souris $C_{57}$Bl/6.

Par ailleurs, les 55185 RP et 59451 RP ne manifestent pas d'effet ou présentent un effet stimulant sur la production de cellules formatrices de plages [technique de N.K. Jerne et A.A. Nodin, Science, 140, 405 (1963)]. Ils sont sans effet sur la croissance de la leucémie P388 chez la souris lorsqu'ils sont administrés à des doses comprises entre 12,5 et 50 mg/kg i.p. pendant 4 jours.

En thérapeutique humaine, les 55185 RP et 59451 RP sont particulièrement utiles pour lutter contre les rejets de greffes d'organes ou de tissus et pour le traitement des maladies auto-immunes telles que les rhumatismes, le diabète insulino-dépendant, la sclérose en plaque, les myasthénies, le lupus, le psoriasis ou la maladie de Crohn.

En thérapeutique humaine, les doses dépendent de l'effet recherché et de la durée du traitement. Elles sont généralement comprises entre 0,5 et 100 mg/kg/jour par voie orale et comprises entre 0,1 et 20 mg/kg/jour par voie parentérale pour un adulte.

D'une manière générale, le médecin déterminera la posologie qu'il estime la plus appropriée en fonction de l'âge, du poids et de tous les autres facteurs propres au sujet à traiter.

Les compositions pharmaceutiques contenant le 55185 RP et/ou le 59451 RP à l'état pur ou en présence d'un diluant ou d'un enrobage constituent un autre objet de la présente invention. Ces compositions peuvent être utilisées par voie orale, rectale ou parentérale.

Comme compositions solides pour administration orale peuvent être utilisés de comprimés, des pilules, des poudres ou des granulés. Dans ces compositions, le produit actif selon l'invention est mélangé à un ou plusieur diluants inertes tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autre que les diluants, par exemple des produits mouillants, édulcorants ou aromatisants.

Les compositions pour administration parentérale peuvent être des solutions stériles aqueuses ou non aqueuses, des supsensions ou des émulsions. Comme solvant ou véhicule, on peut employer le propylèneglycol, le polyéthylèneglycol, les huiles végétales, en particulier l'huile d'olive, et les esters organiques injectables, par exemple l'oléate d'éthyle.

Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent être également préparées sous forme de compositions solides stériles qui peuvent être dissoutes au moment de l'emploi dans un milieu stérile injectable.

Les compositions pour administration rectale sont des suppositoires qui peuvent contenir, outre le principe actif, des excipients tels que le beurre de cacao ou des triglycérides semi-synthétiques.

Les exemples suivants, donnés à titre non limitatif, illustrent des compositions selon l'invention.

EXEMPLE A

On prépare selon la technique habituelle des comprimés dosés à 25 mg de 55185 RP ayant la composition suivante :

- 55185 RP 0,025 g
- amidon 0,090 g
- silice précipitée 0,030 g
- stéarate de magnésium 0,005 g

EXEMPLE B

On prépare selon la technique habituelle une solution administrable par voie parentérale ayant la compositions suivante :

- 55185 RP 0,5 g
- soluté injectable 5 cm3

EXEMPLE C

On prépare selon la technique habituelle des comprimés dosés à 25 mg de 59451 RP ayant la composition suivante :

- 59451 RP 0,025 g
- amidon 0,090 g
- silice précipitée 0,030 g
- stéarate de magnésium 0,005 g

EXEMPLE D

On prépare selon la technique habituelle une solution administrable par voie parentérale ayant la composition suivante :

- 59451 RP 0,5 g
- soluté injectable 5 cm3

**Revendications**

1. - Nouvelles substances immuno-suppressives, désignées par les numéros 55185 RP et 59451 RP, qui sont des cyclodepsipeptides répondant à la formule générale :

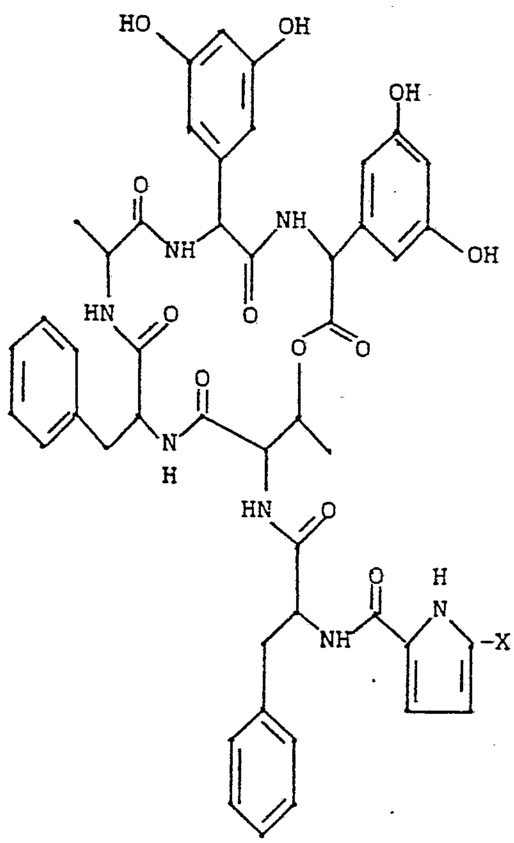

dans laquelle X représente un atome de chlore (55185 RP) ou un atome d'hydrogène (59451 RP).

2 - Nouvelle substance immuno-suppressive désignée par le numéro 55185 RP caractérisée en ce que :

- c'est une poudre amorphe blanche à jaune pâle, soluble dans le méthanol, l'éthanol, l'acétate d'éthyle, l'acétone, l'acétonitrile, le diméthylformamide, le diméthylsulfoxyde, insoluble dans l'eau, l'éther éthylique et l'hexane,
- sa formule brute est $C_{46}H_{46}ClN_7O_{12}$
- sa composition élémentaire est voisine de :
C % = 58,29 H % = 5,30 Cl % = 3,84 N % = 10,13 et 0 % = 22,57
- son point de fusion est supérieur à 300° C
- son pouvoir rotatoire, déterminé dans le méthanol, est
$[\alpha]^{20}_{D}$ = +20,5° ± 1,4°
- son spectre ultra-violet dans le méthanol présente un épaulement à 230 nm et un maximum d'absorption à 274 nm ($E^{1\%}_{1cm}$ = 219 ; ε = 20240)
- son spectre infra-rouge, déterminé à partir de comprimés en mélange avec KBr, présente les bandes d'absorption caractéristiques suivantes :
3380, 3300, 3090, 3060, 3030, 2980, 2940, 2880, 2700, 2500, 2340, 2160, 1950, 1880, 1740, 1680, 1655, 1625, 1605, 1555, 1520, 1500, 1455, 1440, 1425, 1405, 1380, 1340, 1310, 1285, 1215, 1205, 1160, 1120, 1080, 1060, 1050, 1030, 1010, 1000, 980, 950, 925, 900, 850, 795, 785, 750, 700, 690, 635, 625, 605, 580, 525, 510 495, 455, 450 et 355 $cm^{-1}$
- en chromatographie ascendante sur couche mince de gel de silice avec comme solvant un mélange dichloro-1,2 éthane-méthanol (80-20 en volumes), il a un Rf de 0,5.

3 - Nouvelle substance immuno-suppressive désignée par le numéro 59451 RP caractérisé en ce que :
- c'est une poudre amorphe blanche à jaune pâle, soluble dans le méthanol, l'éthanol, l'acétate d'éthyle, l'acétone, l'acétonitrile, le diméthylsulfoxyde, insoluble dans l'eau, l'éther éthylique et l'hexane,
- sa formule brute est $C_{46}H_{47}N_7O_{12}$
- sa composition élémentaire est voisine de :
C % = 62,09 H % = 5,32 O % = 21,57 N % = 11,02
- son point de fusion est de 275-280° C (décomposition)
- son pouvoir rotatoire, déterminé dans le méthanol, est :
$[\alpha]_D$ = +24,3° ± 1°
- son spectre ultra-violet dans le méthanol présente un maximum d'absorption à 268 nm (ε = 19864)
- son spectre infra-rouge, déterminé à partir de comprimés en mélange avec KBr, présente les bandes d'absorption caractéristiques suivantes :
3360, 3480, 3400, 3280, 3060, 3040, 2980, 2940, 2880, 2680, 2520, 2160, 2060, 1950, 1880, 1740, 1675, 1655, 1640, 1625, 1605, 1555, 1530, 1500, 1455, 1445, 1415, 1380, 1340, 1310, 1295, 1275, 1245, 1230, 1190, 1170, 1145, 1110, 1090, 1070, 1060, 1040, 1030, 1000, 990, 970, 960, 930, 920, 900, 885, 870, 850, 830, 785, 750, 740, 700, 690, 645, 620, 605, 585, 550, 520, 505, 470, 445, 400 et 330 $cm^{-1}$.

4 - Procédé de préparation des nouvelles substances selon l'une des revendications 1, 2 ou 3 caractérisé en ce que l'on cultive de façon aérobie Streptomyces sp. S-16328 (CBS 162.86) ou ses mutants producteurs, sur un milieu classique convenable et dans des conditions habituelles pour ce genre de culture puis sépare les 55185 RP et 59451 RP et les purifie.

5 - Procédé de préparation de la substance 59451 RP caractérisé en ce que l'on hydrogénolyse la substance 55185 RP au moyen d'hydrogène en présence d'un catalyseur à une température voisine de 20° C.

6 - Procédé selon la revendication 5 caractérisé en ce que le catalyseur est le palladium sur charbon.

7 - Procédé selon l'une des revendications 5 ou 6 caractérisé en ce que l'on opère en présence d'un accepteur d'acide.

8 - Procédé selon la revendication 7 caractérisé en ce que l'accepteur d'acide est la magnésie.

9 - Composition pharmaceutique caractérisée en ce qu'elle contient une quantité suffisante de la substance 55185 RP et/ou 59451 RP selon l'une des revendications 1, 2 ou 3, à l'état pur ou en présence d'un diluant ou d'un enrobage pharmaceutiquement acceptable.

Revendications pour les Etats contractants suivants: Autriche, Espagne, Grèce

1 - Procédé de préparation de nouvelles substances immuno-suppressives, désignées par les numéros 55185 RP et 59451 RP, qui sont des cyclodepsipeptides répondant à la formule générale :

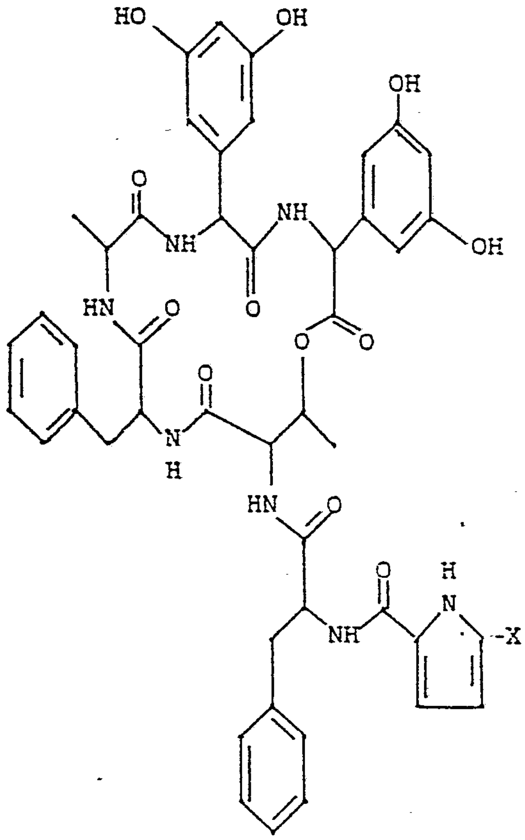

dans laquelle X représente un atome de chlore (55185 RP) ou un atome d'hydrogène (59451 RP), caractérisé en ce que l'on cultive de façon aérobie Streptomyces sp. S-16328 (CBS 162.86) ou ses mutants producteurs, sur un milieu classique convenable et dans des conditions habituelles pour ce genre de culture puis sépare les 55185 RP et 59451 RP et les purifie.

2 - Procédé de préparation de la substance 59451 RP caractérisé en ce que l'on hydrogénolyse la substance 55185 RP au moyen d'hydrogène en présence d'un catalyseur à une température voisine de 20° C.

3 - Procédé selon la revendication 2 caractérisé en ce que le catalyseur est le palladium sur charbon.

4 - Procédé selon l'une des revendications 2 ou 3 caractérisé en ce que l'on opère en présence d'un accepteur d'acide.

5 - Procédé selon la revendication 4 caractérisé en ce que l'accepteur d'acide est la magnésie.

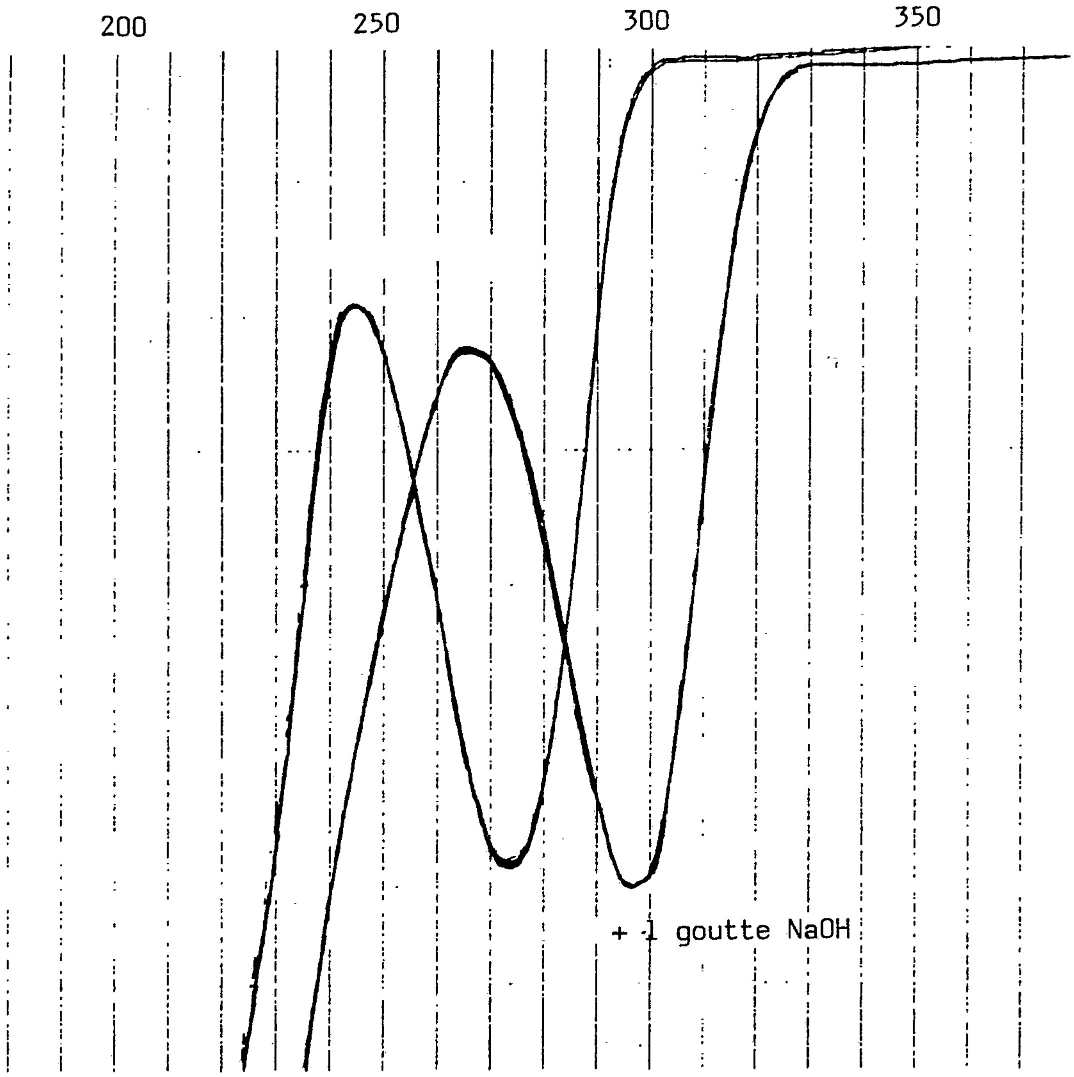


FIGURE 1

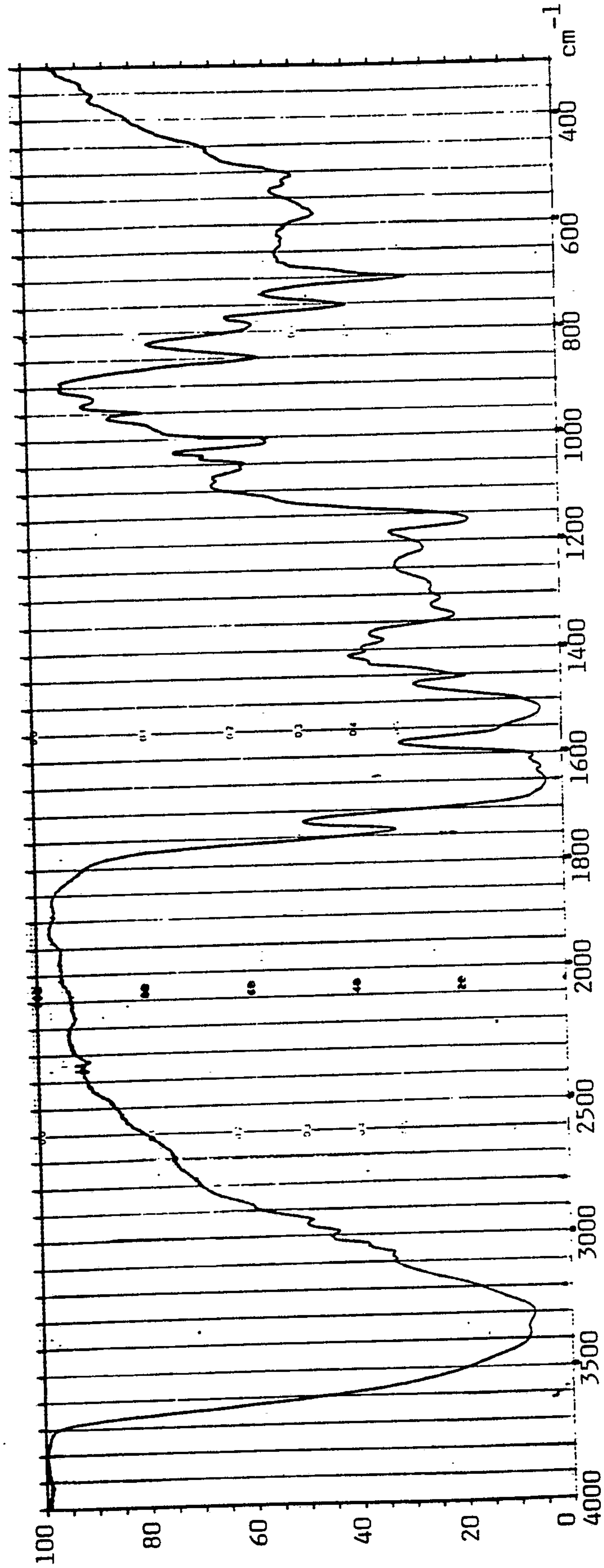
cm-1
400
600
800
1000
1200
1400
1600
1800
2000
2500
3000
3500
4000
100
80
60
40
20
0

FIGURE 2

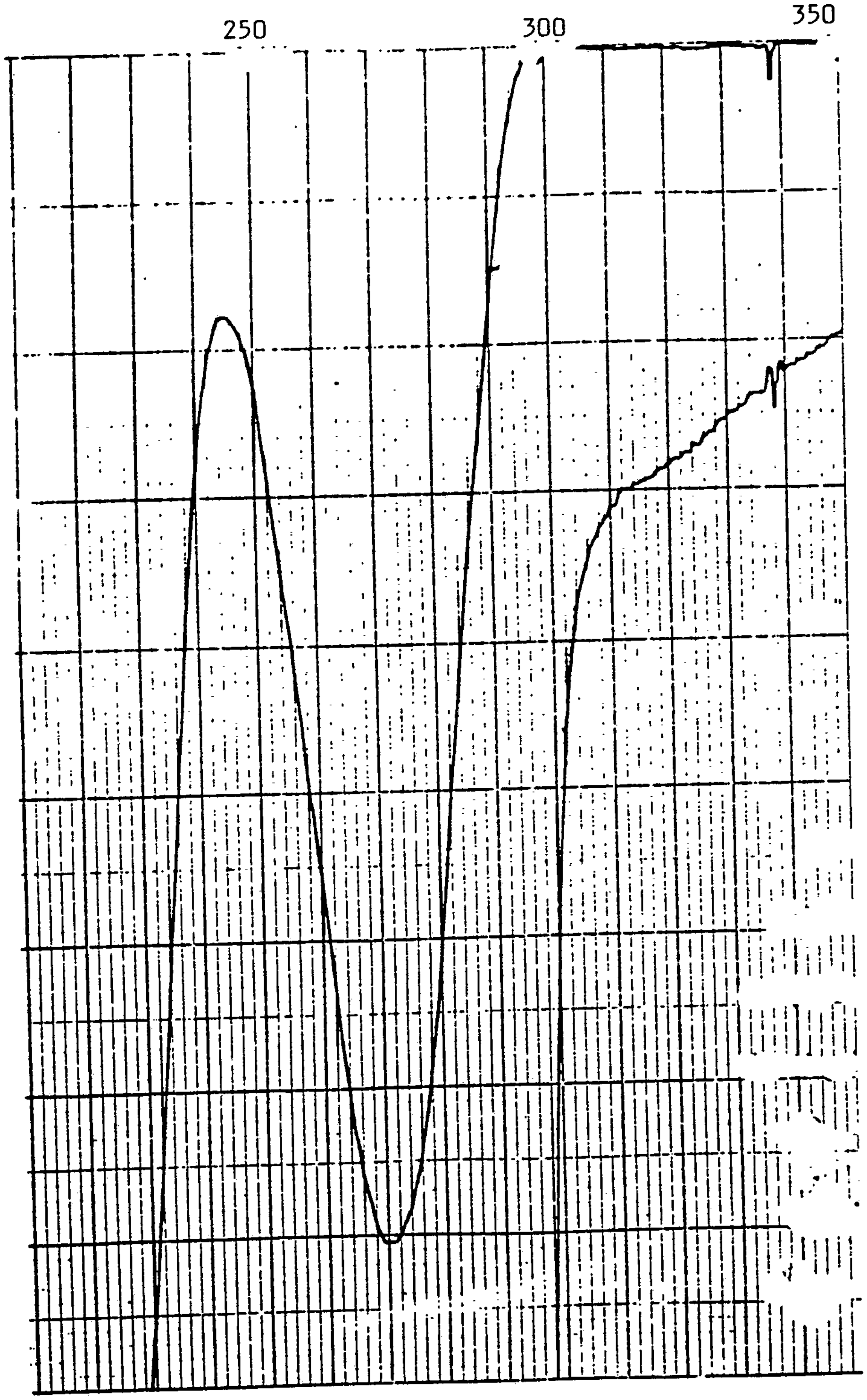


FIGURE 3

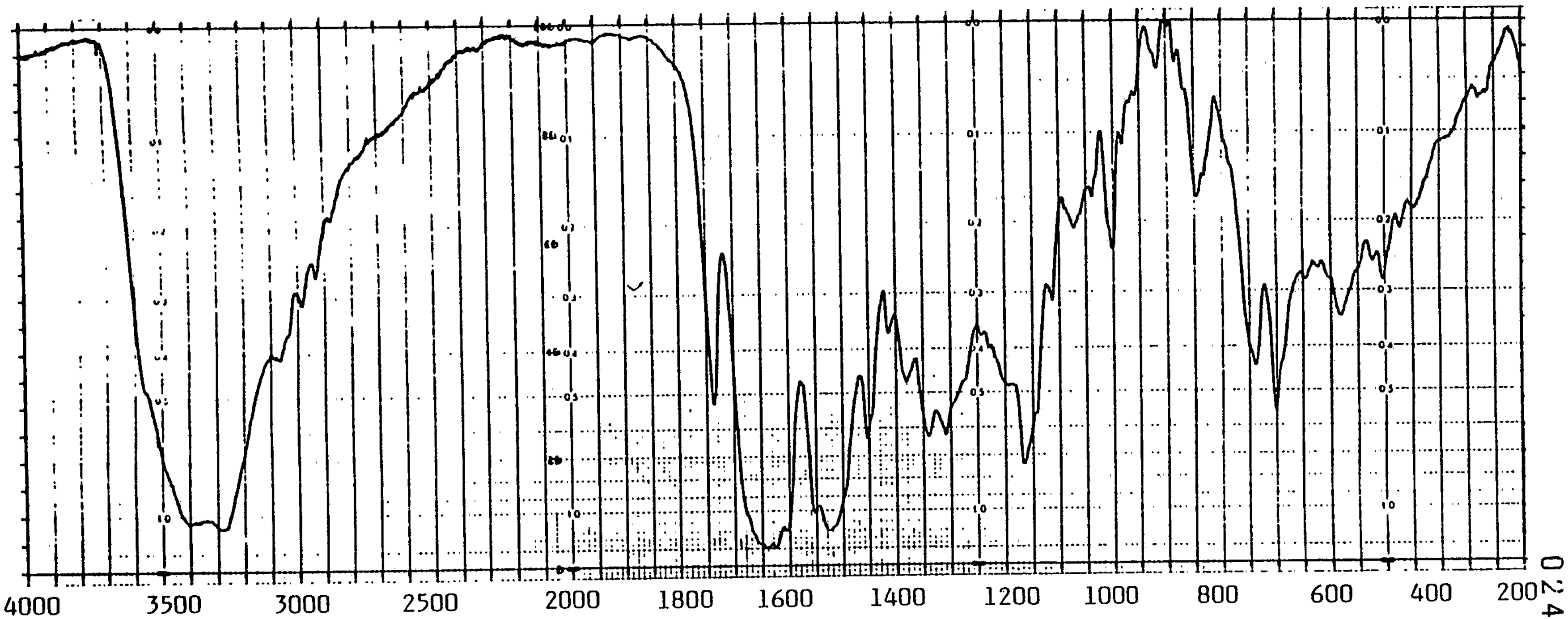
4000
3500
3000
2500
2000
1800
1600
1400
1200
1000
800
600
400
200

FIGURE 4

Office européen des brevets

# RAPPORT DE RECHERCHE EUROPEENNE

Numéro de la demande

EP 87 40 1141

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| A | US-A-4 355 112 (OKUMURA)<br>* Page de garde, colonnes 9-10 *<br>----- | 1 | C 12 P 21/04<br>C 07 K 7/06<br>A 61 K 37/02 //<br>(C 12 P 21/04<br>C 12 R 1:465) |

DOMAINES TECHNIQUES RECHERCHES (Int. Cl.4)

C 12 P 21/00
C 07 K 7/00
C 12 R 1/00
A 61 K 37/00

Le présent rapport de recherche a été établi pour toutes les revendications

| Lieu de la recherche | Date d'achèvement de la recherche | Examinateur |
|---|---|---|
| LA HAYE | 27-08-1987 | RAJIC M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant